# EUROPEAN PATENT SPECIFICATION

(11) **EP 1 287 842 B1**
(45) Date of publication and mention of the grant of the patent: **18.01.2006**
(21) Application number: 02018685.4
(22) Date of filing: 21.08.2002
(51) Int. Cl.: A61M 5/50, A61M 5/32

(54) **Safety device for a safety syringe**
Sicherheitsvorrichtung für eine Sicherheitsspritze
Dispositif de sécurité pour une seringue de sécurité

(30) Priority: 27.08.2001 CN 01253289
(43) Date of publication of application: 05.03.2003
(73) Proprietor: Lee, Tun-Chi, Hsintien City, Taipei Hsien, (TW); Chen, Tao-Sun, Taipei (TW); Kuo, Man-Yun, Taipei (TW); Huang, Ting-Hsien, Taipei (TW); Chen, Wen-Lin, Keelung (TW); Jiun-Hsin Investment Co., Ltd.,, Taipei (TW); Syriteck Medical Devices Co., Ltd, Taichung (TW)
(72) Inventor: Shen, Ting, Shanghai (CN)
(74) Representative: LOUIS, PÖHLAU, LOHRENTZ

(56) References cited:
- EP-A- 0 979 660
- EP-A- 1 216 719
- DE-U- 20 116 321
- DE-U- 29 818 132
- FR-A- 2 689 766
- US-A- 5 328 484
- US-A- 6 093 171

## Description

### 1. Field of the Invention

The present invention relates to a safety syringe, and more particularly to a safety syringe according to the preamble of the claim which is able to prevent accidental engagement of a hook to a needle hub and to satisfactory pull the needle hub back into the barrel when required so that the syringe is able to be disposed of safely.

### 2. Description of Related Art

Numerous patents discussing different structures and means of preventing accidental damage to paramedic personnel by used syringes have already been introduced to the market. Reviewing the patents, most of them concern the means of how the needle is retracted into the barrel. However, in most of the information provided in the available patents, if the user is not careful when trying to receive medicine in the barrel, the plunger prematurely securely engages with the needle hub such that the syringe is no longer useful in injection.

The most related patents are DE-U-29818132 and US-A-5,328,484. The needle and the solid cone in the DE-U-29818132 are able to be pulled down in the barrel. However, this prior art does not have the ability to safeguard patients' safety. The arrangement of the hook and the needle hub can only move the needle hub in a direction horizontal to a central axis of the plunger so that the second frontward movement of the plunger after engagement between the plunger and the needle hub will extend the needle together with the needle hub out of the plunger intact without any damage. Therefore, an ill-intentioned person may use this design defect to reuse the already used needle and thus patients' safety is endangered.

With reference to the US-A-5,328,484, the description repeatedly mentions that an elastomeric plug is provided in the syringe and has two different types, one is that the elastomeric plug is rigid or hard to resist the downward movement of the plunger so that the needle is able to be driven to penetrate said plug and to be advanced out of the syringe, and the other one is that the elastomeric plug is soft such that when the plunger is moved toward the needle carrier, the hook is able to engage with the flange. Hence a rearward movement of the plunger is able to retract the needle back into the syringe for damaging the needle afterward. Besides, the elastomeric plug the ring is located in the needle carrier. The plunger has to have a plate provided with a skirt extending downward from a bottom face of the plate such that when the plunger is moving downward to the needle carrier, the skirt is able to press on the elastomeric plug. Thereafter, the needle is able to penetrate through a member as depicted in Figs. 1 and 2 of the mentioned US-A-5,328,484. The ring first serves as a hard member so that when the plate engages with the ring, the needle penetrates through the member. Then after the ring is softened due to the soaking by the liquid in the syringe, the plunger's forward movement toward the needle carrier enables the hook to engage with the needle carrier such that the needle carrier as well as the needle is pulled back into the syringe for damage. However, it is observed that after the ring is softened due to the liquid, the forward movement of the plunger causes the hook to engage with the needle carrier. Therefore there is no room left in the barrel for the operator to disperse the air inside the barrel, which requires the plunger to move forward to force the air out from the bore of the needle. In this case, the patient, the person receiving the injection, may be exposed to a risk of having a serious consequence due to the entrance of air into the vein.

To overcome the shortcomings, the present invention tends to provide an improved safety syringe to mitigate and obviate the aforementioned problems.

The primary objective of the present invention is to provide an improved safety syringe so that prior to giving the injection the user is able to readily pull the needle hub back into the barrel without worrying that the needle hub is accidentally retracted into the barrel.

The foregoing objective will be accomplished by a syringe with the features of claim 1.

Other objects, advantages and novel features of the invention will become more apparent from the following detailed description when taken in conjunction with the accompanying drawings.
In The Drawings
Fig. 1 is an exploded perspective view of showing the parts of the syringe of the present invention;
Fig. 2 is a cross sectional view showing an assembly among the barrel, the needle hub and the plunger;
Fig. 3 is a cross sectional view showing the hook engages with the flange of the needle hub;
Fig. 4 is a cross sectional view showing that the needle hub is retracted back into the barrel by the rearward movement of the plunger; and
Fig. 5 is a schematic view showing that the needle of the needle hub is broken by the forward movement of the plunger.

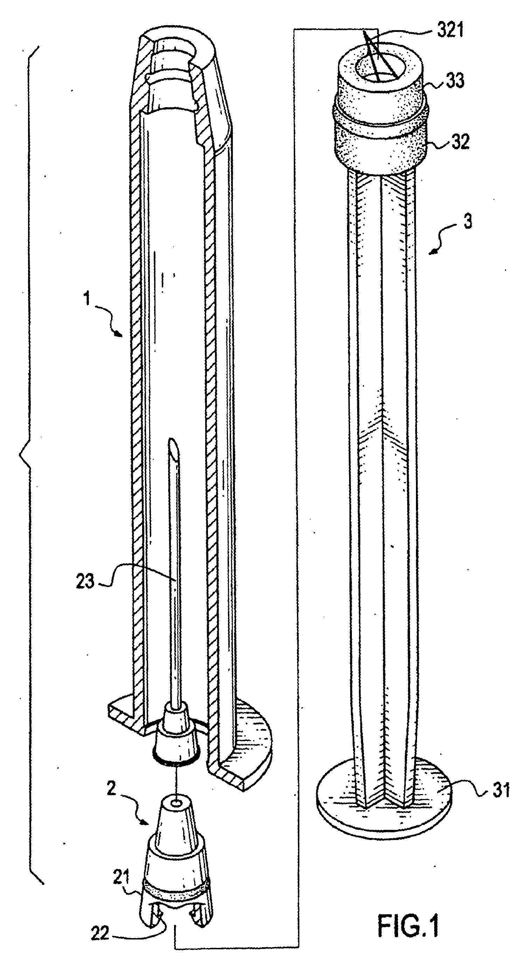

With reference to Fig. 1, a safety syringe has a barrel (1), a needle hub (2) and a plunger (3).

The barrel (1) is hollow inside to receive therein the needle hub (2) and the plunger (3).

The needle hub (2) is detachably received in the barrel (1) and has a hollow cone (21) and a flange (22) formed on an inner periphery of the hollow cone (21) of the needle hub (2). A needle (23) is replaceably mounted on top of the needle hub (2) so that when necessary, the user is able to replace a damaged needle (23) before use.

The plunger (3) has a thumb push (31) formed on a first distal end of the plunger (3) and a stop (32) formed on a second distal end of the plunger (3) and having a hook (321) integrally formed on a top face of the stop (32) to correspond to the flange (22) of the hollow cone (21). The plunger (3) further has a spacer (33) integrally formed on the top face of the stop (32). The spacer (33) is made of a material with resilience such that the spacer (33) is deformable. Furthermore, the hook (321) extends out of the spacer (33) from the stop (32).

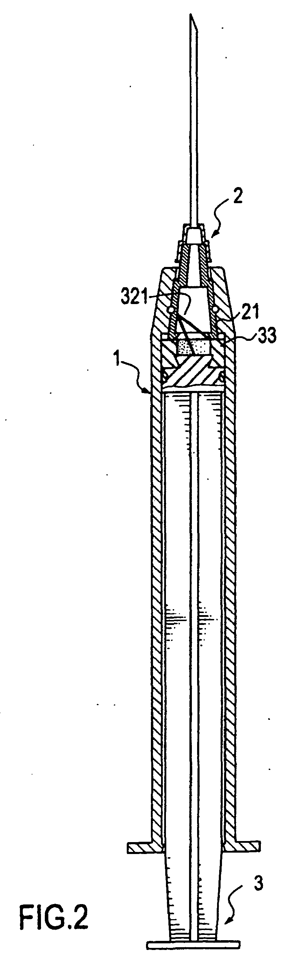

With reference to Fig. 2, when the safety syringe of the present invention is in assembly, the needle hub (2) is first inserted into the barrel (1) so as to secure relative position of the needle hub (2) to the barrel (1). Meantime, the plunger (3) is also received in the barrel (1) to be ready to receive medicine in the barrel (1). From Fig. 2 it is noted that before the syringe of the present invention is in use, the spacer (33) is in engagement with a bottom face of the hollow cone (21) of the needle hub (2) and a free end of the hook (321) is not received in the hollow cone (21), thereby the user can safely use the syringe to receive medicine in the barrel (1) without worrying that the hook (321) might be accidentally in engagement with the needle hub (2) to disable the syringe.

Furthermore, the hook (321) is preferably made of a metal. The hook (321) is bent so that a free end of the hook (321) is resilient relative a distal end securely formed with the stop (32).

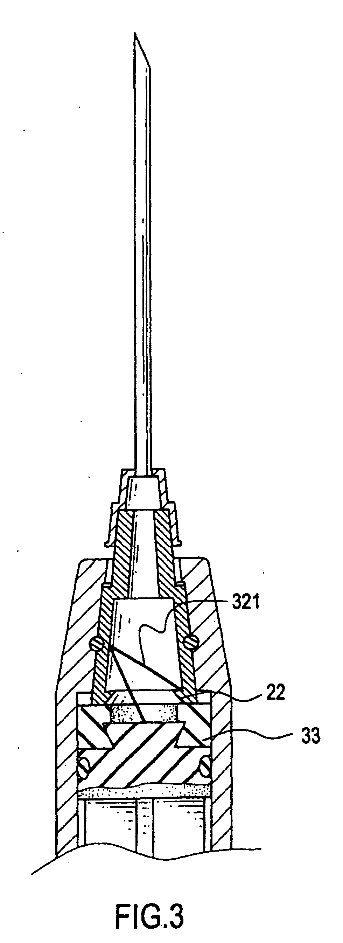

With reference to Fig. 3, when the medicine inside the barrel (1) is ejected out of the barrel (1), the user pushes the plunger (3) all the way into the barrel (1) to force the spacer (33) to deform such that the hook (321) is received in the hollow cone (21). Because of the resilience of the hook (321), the hook (321) is able to engage with the flange (22) of the hollow cone (21). Thereafter the user is ready to pull the needle hub (2) back into the barrel (1).

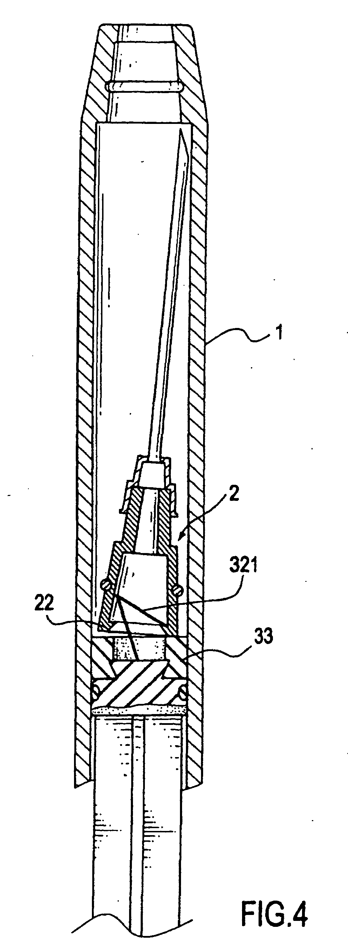

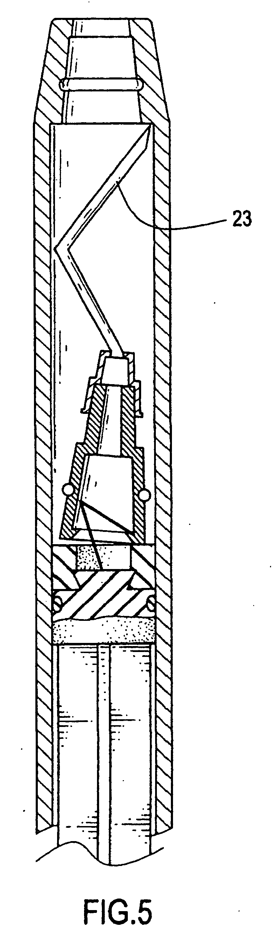

With reference to Fig. 4, after the needle hub (2) is retracted in the barrel (1), because the free end of the hook (321) is in engagement with one point of the flange (22), the needle hub (2) is inclined relative to the barrel (1). When the needle hub (2) is inclined to and fully received in the barrel (1), a forward movement of the plunger (3) together with the needle hub (2), as shown in Fig. 5, the needle (23) is bent inside the barrel (1) such that the safety syringe of the present invention is able to be disposed of safely.

It is thus noted that the safety device used in the safety syringe of the present invention includes the spacer (33) formed on a top face of the stop (32), the hook (321) formed with the stop (32) and extending out from the spacer (33) and a flange (22) formed on the inner periphery of the hollow cone (21) of the needle hub (2).

With such an arrangement of the safety device of the present invention, the user is able to safely use the syringe to perform an injection and readily pull the needle hub (2) together with the needle (23) back into the barrel (1).

It is to be understood, however, that even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A safety syringe having a barrel (1), a needle hub (2) movably received in the barrel (1) and having a needle (23) securely attached to a distal end of the needle hub (2) and a plunger (3) slidably received in the barrel (1) and having a thumb press (31) formed on a proximal end of the plunger (3) and a stop (32) formed on a distal end of the plunger (3),
the needle hub (2) has a hollow cone (21) with a flange (22) formed on an inner periphery of the hollow cone (21);
the plunger has a hook (321) integrally formed on a top face of the stop (32) to correspond to the flange (22) of the needle hub (2) so that when the hook (321) engages with the flange (22) of the needle hub (2), the needle hub (2) is able to be pulled back into the barrel (1) in a direction offset to a central axis of the plunger (3) by a rearward movement of the plunger (3) and the needle (23) is damaged by a forward movement of the plunger (3) after the needle hub (2) is entirely received in the barrel (1), and a spacer (33) formed on the top face of the stop (32)
**characterized in that**
the spacer (33) is adapted to detachably engage with a bottom face of the hollow cone (21) to keep the hook (321) from engagement with the flange (22), the spacer (33) being made of a resilient material such that the spacer can be deformed to an extent so that the hook (321) can engage with the flange (22) by which engagement the needle hub (2) can be pulled back into the barrel (1).

## Patentansprüche

1. Sicherheitsspritze mit einem Zylinder (1), einer Nadelbuchse (2), die in dem Zylinder (1) beweglich aufgenommen ist und eine Nadel (23) besitzt, die an einem distalen Ende der Nadelbuchse (2) sicher befestigt ist, und einem Tauchkolben (3), der in dem Zylinder (1) gleitend aufgenommen ist und einen Daumenpressabschnitt (31), der an einem proximalen Ende des Tauchkolbens (3) ausgebildet ist, und einen Anschlag (32), der an einem distalen Ende des Tauchkolbens (3) ausgebildet ist, aufweist,
wobei die Nadelbuchse (2) einen Hohlkonus (21) mit einem an einem inneren Umfang des Hohlkonus (21) ausgebildeten Flansch (22) aufweist;
wobei der Tauchkolben einen an einer oberen Fläche des Anschlags (32) einteilig ausgebildeten Haken (321) besitzt, der dem Flansch (22) der Nadelbuchse (2) entspricht, so dass dann, wenn der Haken (321) mit dem Flansch (22) der Nadelbuchse (2) in Eingriff ist, die Nadelbuchse (2) in einer von der Mittelachse des Tauchkolbens (3) versetzten Richtung durch eine Rückwärtsbewegung des Tauchkolbens (3) in den Zylinder (1) zurückgezogen werden kann und die Nadel (23) durch eine Vorwärtsbewegung des Tauchkolbens (3) beschädigt wird, nachdem die Nadelbuchse (2) vollständig in dem Zylinder (1) aufgenommen worden ist, wobei der Tauchkolben ferner einen an der oberen Fläche des Anschlags (32) ausgebildeten Abstandshalter (33) besitzt,
**dadurch gekennzeichnet, dass**
der Abstandshalter (33) so beschaffen ist, dass er mit einer Bodenfläche des Hohlkonus (21) in lösbarem Eingriff ist, um den Haken (321) an einem Eingriff mit dem Flansch (22) zu hindern, wobei der Abstandshalter (33) aus einem elastischen Material hergestellt lst, derart, dass der Abstandshalter in einem Ausmaß verformt werden kann, dass der Haken (321) mit dem Flansch (22) in Eingriff gelangen kann, wobei durch diesen Eingriff die Nadelbuchse (2) in den Zylinder (1) zurückgezogen werden kann.

## Revendications

1. Une seringue de sécurité, du type comprenant un corps (1), un porte-aiguille (2), logé mobile dans le corps (1), une aiguille (23) fixée à l'extrémité distale du porte-aiguille (2), un piston (3), monté coulissant dans le corps (1) et comportant un poussoir (31) créé sur l'extrémité proximale du piston (3) et une butée (32), créée sur l'extrémité distale du piston (3),
le porte-aiguille (2) comportant un cône creux (21), un rebord (22) étant créé sur la périphérie intérieure du cône creux (21) ;
le piston présentant un crochet (321) réalisé en une seule pièce avec la face supérieure de la butée (32), de manière à correspondre au rebord (22) du porte-aiguille (2), de sorte que, quand le crochet (321) rencontre le rebord (22) du porte-aiguille (2), le porte-aiguille (2) peut être ramené dans le corps (1) dans une direction décalée par rapport à l'axe central du piston (3), sous l'effet d'un déplacement vers l'arrière du piston (3), et l'aiguille (23) est endommagée par un déplacement vers l'avant du piston (3) après que le porte-aiguille (2) ait été entièrement enfoncé dans le corps (1), ainsi qu'une cale d'espacement (33) créée sur la face supérieure de la butée (32),
**caractérisée en ce que**
la cale d'espacement (33) est prévue pour rencontrer de manière séparable la face inférieure du cône creux (21), de manière à empêcher le contact du crochet (321) avec le rebord (22), la cale d'espacement (33) étant réalisée en un matériau élastique, de sorte que la cale d'espacement peut être déformée à un degré tel que le crochet (321) puisse coopérer avec le rebord (22), grâce à quoi le porte-aiguille (2) peut être ramené dans le corps (1).
